(19) **Europäisches Patentamt / European Patent Office / Office européen des brevets**

(11) **EP 2 994 091 B1**

(12) **FASCICULE DE BREVET EUROPEEN**

(45) Date de publication et mention de la délivrance du brevet:
**13.02.2019 Bulletin 2019/07**

(21) Numéro de dépôt: **14726424.6**

(22) Date de dépôt: **06.05.2014**

(51) Int Cl.:
*A61K 8/365* (2006.01)     *A61K 8/58* (2006.01)
*A61K 8/27* (2006.01)      *A61Q 5/00* (2006.01)
*A61Q 7/00* (2006.01)      *A61K 8/36* (2006.01)
*A61K 8/92* (2006.01)      *A61K 8/46* (2006.01)
*A61K 8/97* (2017.01)      *A61Q 5/06* (2006.01)
*A61K 8/67* (2006.01)      *A23L 33/12* (2016.01)

(86) Numéro de dépôt international:
**PCT/IB2014/061245**

(87) Numéro de publication internationale:
**WO 2014/181257 (13.11.2014 Gazette 2014/46)**

(54) **ASSOCIATION D'ACIDE PETROSELINIQUE ET DE ZINC POUR UNE ADMINISTRATION PAR VOIE ORALE POUR LUTTER CONTRE LE VIEILLISSEMENT DES CHEVEUX**

KOMBINATION VON PETROSELINSÄURE UND ZINK ZUR ORALEN VERABREICHUNG ZWECKS ALTERUNGSSTEUERUNG DER HAARE

COMBINATION OF PETROSELINIC ACID AND ZINC FOR ORAL ADMINISTRATION FOR HAIR AGING CONTROL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.05.2013 FR 1354187**

(43) Date de publication de la demande:
**16.03.2016 Bulletin 2016/11**

(73) Titulaire: **NUTRICOS Technologies
92117 Clichy Cedex (FR)**

(72) Inventeurs:
• **MAHE, Yann
F-91700 Sainte Genevieve Des Bois (FR)**
• **BRU, Carole
F-92400 Courbevoie (FR)**

(74) Mandataire: **Nony
11 rue Saint-Georges
75009 Paris (FR)**

(56) Documents cités:
EP-A1- 1 013 178     EP-A1- 1 932 509
WO-A2-02/07700      WO-A2-2008/071897
CN-A- 102 293 312   US-A1- 2005 175 565

• FRDRIC DESTAILLATS ET AL: "Identification of 6-monounsaturated fatty acids in human hair and nail samples by gas-chromatographymass-spectrometry using ionic-liquid coated capillary column", JOURNAL OF CHROMATOGRAPHY, ELSEVIER SCIENCE PUBLISHERS B.V, NL, vol. 1218, no. 52, 21 octobre 2011 (2011-10-21), pages 9384-9389, XP028338533, ISSN: 0021-9673, DOI: 10.1016/J.CHROMA.2011.10.095 [extrait le 2011-11-15]
• SINGH V ET AL: "Availability of essential trace elements in Indian cereals, vegetables and spices using INAA and the contribution of spices to daily dietary intake", FOOD CHEMISTRY, ELSEVIER LTD, NL, vol. 94, no. 1, 1 janvier 2006 (2006-01-01), pages 81-89, XP027989042, ISSN: 0308-8146 [extrait le 2006-01-01]

**Description**

La présente invention se rapporte au domaine du soin des fibres kératiniques, plus particulièrement des cheveux. En particulier, elle vise à proposer une association utile pour améliorer la qualité de la chevelure et pour lutter contre le vieillissement des fibres kératiniques et plus particulièrement capillaires, provoquant en particulier un affinement des fibres kératiniques ou plus globalement des cheveux.

Comme cela apparaitra par la suite, il est plus particulièrement question dans le cadre de la présente invention de la lutte contre le vieillissement des cheveux chez l'homme de plus de 30 ans ou chez la femme.

Les cheveux sont produits dans des follicules pileux formés de gaines épithéliales d'origine épidermique et d'un bulbe pileux contenant des kératinocytes bulbaires en état de division permanent pendant les phases de croissances du cheveu. Le cheveu est constitué principalement de 85-90 % de protéines.

Avoir des cheveux sains et vigoureux tout au long de la vie est recherché par la plupart des femmes et des hommes.

Ainsi c'est un premier objectif de l'invention que de proposer une solution aux hommes et femmes de tout âge pour améliorer la qualité de leur chevelure.

En particulier, les hommes de plus de 30 ans et les femmes sont soumis à un vieillissement de leurs fibres kératiniques, en particulier capillaires. Ils peuvent ainsi présenter une alopécie liée au vieillissement de la chevelure qui se caractérise notamment par un affinement et une perte de matière globale de la chevelure et d'un éclaircissement progressif.

Ceci se distingue par certains aspects de l'alopécie dite androgénétique dont la transmission est par définition héréditaire, dont la progression est rapide et affecte initialement le front et les tempes et, en raison du rôle des androgènes, se signale très tôt après la puberté, principalement chez l'homme jeune.

De récents travaux ont mis en évidence chez les individus plus âgés, et indifféremment chez les hommes et les femmes, une forme d'alopécie plus longue à s'installer dans le temps mettant en jeu la contribution d'une forme latente et silencieuse d'inflammation (Pro-inflammatory cytokine cascade in human plucked hair Mahé et al. Skin Pharmacol 1996, 366-375), dite à bas bruit, et appelée microinflammation (Mahé et al. Androgenetic alopecia and micro inflammation Int. J. Dermatol., 2000, 39, 576-584 et par Trueb, Clinical intervention in aging hair, 2006 :1(2) 121-129) comme contributeur de l'involution progressive des follicules pileux et de la miniaturisation du follicule pileux.

Ce phénomène dit de microinflammation du follicule pileux a été identifié et décrit dans Mahé et al. (International Journal of Dermatology, 2000, 39, 576-584) comme étant une inflammation lente, subtile et indolore, qui n'entraîne ni rougeurs, démangeaisons ou chaleurs, ni aucun des troubles d'ordre thérapeutique habituellement associés à une inflammation du scalp.

Bien que pouvant faire intervenir des infiltrats cellulaires inflammatoires lymphocytaires ou monocytaires perifolliculaires, elle se distingue nettement de l'inflammation dite classique, pouvant avoir lieu au niveau du derme ou du scalp, qui se manifeste de manière bien plus gênante, voire douloureuse, et qui a des conséquences bien plus destructrices sur le cuir chevelu et sur la fibre capillaire. Notamment, elle se distingue de l'*alopecia areata* liée à un trouble immuno-inflammatoire établi et qui aboutit à une chute massive et parfois localisée et souvent spontanément réversible des cheveux, affectant indifféremment des hommes, des femmes et des enfants, ce qui permet d'exclure a priori la contribution des androgènes dans sa physiopathologie.

La microinflammation à bas bruit contribue au contraire à un vieillissement plus progressif et intrinsèque du scalp et des cheveux. Comme indiqué précédemment, la microinflammation périfolliculaire et folliculaire selon l'invention peut entrainer, outre un affinement des cheveux, une chute des cheveux, et aboutir à une alopécie plutôt diffuse voire centrée sur le vertex, en particulier chez l'homme de plus de 30 ans ou chez la femme, par opposition à l'alopecie androgenetique qui démarre au niveau du vertex et du front et affecte principalement les hommes jeunes.

Le vieillissement du cheveu, caractérisé par cette microinflammation, est en effet un phénomène naturel qui se manifeste progressivement et plus ou moins tôt selon les individus. Cette microinflammation est difficilement perceptible en surface et a notamment pour conséquence le déclenchement de perturbations dermiques perifolliculaires conduisant à un épaississement de la gaine interne du cheveu et à sa miniaturisation progressive au fur et à mesure des cycles successif de régénération d'un nouveau follicule fonctionnel. On constate ainsi un affinement du cheveu, un cheveu moins épais, moins résistant à la traction et à la casse, et une diminution du volume de la chevelure globale. En outre, le scalp devient visible par endroits avec un net dégarnissement du vertex, sans pour autant systématiquement affecter fortement les tempes ou le front. Parfois même, cette alopécie diffuse, tardive et à évolution lente peut prendre le relais ou renforcer et accentuer les effets d'une phase initiale d'alopécie androgénétique. En outre, le vieillissement du cheveu peut également se caractériser par une usure et une altération de la fibre capillaire se traduisant par des cheveux dits fragilisés/sensibilisés, ou bien encore des cheveux secs.

Ainsi, il apparait clairement que cette microinflammation périfolliculaire, qui est représentative du vieillissement intrinsèque du bulbe et des fibres capillaires, n'est à l'origine que de troubles cosmétiques, et notamment de ceux énoncés précédemment. Contrairement à l'inflammation dite classique, dont les troubles associés se situent dans le domaine thérapeutique, la prévention et/ou le traitement de la microinflammation cliniquement silencieuse et à bas bruit du follicule pileux, et ainsi des troubles qui lui sont associés, se situent uniquement dans le domaine cosmétique.

Il existe donc également un besoin de disposer d'actifs, voire d'une association d'actifs, aptes à prévenir et/ou lutter contre la microinflammation des follicules pileux.

En outre, le document WO 2008/071897 décrit une composition comprenant de l'acide pétrosélinique pour l'application topique sur le cheveu, pour le traitement ou la prévention de leur altération, et pour améliorer la qualité des fibres kératiniques.

Pour autant, les consommateurs sont toujours à la recherche d'actifs ou d'association d'actifs plus efficaces pour améliorer la qualité de leur chevelure. En outre, il n'existe pas à ce jour d'actifs spécifiquement adaptés à la prévention et à la lutte contre la microinflammation à bas bruit du follicule pileux et contre l'affinement progressif des cheveux, en particulier chez l'homme de plus de 30 ans ou chez la femme.

La présente invention propose de répondre à ce besoin en proposant une association d'acide pétrosélinique avec du zinc, en particulier des sels de Zn (II), et de préférence complexés par un ou plusieurs (poly)hydroxyacides, tel que le gluconate de zinc, administrée par voie orale.

Comme illustré dans les exemples de la présente demande, les inventeurs ont en effet constaté qu'une association d'actifs conforme à l'invention s'avère apte à augmenter de façon synergique la quantité de lipoxine A4. La lipoxine A4 appartient à la famille des résolvines. Cette famille de composés naturellement produits par l'organisme agit de façon complémentaire aux agents anti-inflammatoires classiques en élevant le seuil de déclenchement d'une réponse inflammatoire dite classique dermatologiquement, et plus particulièrement pour élever le seuil d'apparition des signaux de cette inflammation classique, à savoir les rougeurs, les douleurs et la chaleur.

L'invention a donc pour objet principal l'utilisation cosmétique, par voie orale, d'une association d'actifs comprenant de l'acide pétrosélinique et du zinc, en particulier les sels de Zn (II), et de préférence complexés par un ou plusieurs (poly)hydroxyacides, tel que le gluconate de zinc, pour prévenir et/ou lutter contre la microinflammation à bas bruit des follicules pileux, permettant d'améliorer la qualité de la chevelure, en particulier chez l'homme de plus de 30 ans chez ou la femme, et en particulier pour améliorer la qualité de la fibre capillaire.

De manière préférée selon l'invention, l'utilisation cosmétique par voie orale d'une association d'actifs comprenant de l'acide pétrosélinique et du zinc, en particulier des sels de Zn (II), et de préférence complexés par un ou plusieurs (poly)hydroxyacides, tel que le gluconate de zinc, est caractérisée en ce que l'amélioration de la qualité de la chevelure comprend l'amélioration de la brillance de la chevelure et/ou l'amélioration de la coiffabilité de la chevelure, et/ou sa tenue et son maintien et/ou la diminution de la chute des cheveux et/ou l'amélioration de la croissance de la fibre capillaire, en particulier de fibres capillaires épaisses, et/ou l'amélioration du volume de la chevelure et/ou l'amélioration de la qualité de la fibre capillaire.

De manière encore préférée, l'amélioration du volume de la chevelure comprend l'augmentation et/ou le maintien du diamètre de la fibre capillaire, et/ou l'augmentation de la densité, et/ou la limitation de l'affinement de la fibre capillaire.

De manière également préférée, l'amélioration de la qualité de la fibre capillaire comprend améliorer la résistance à la traction des cheveux, et/ou au coiffage et/ou à la mise en forme des cheveux et/ou prévenir et/ou lutter contre les cheveux mous et/ou cassants et/ou ternes et/ou fourchus et/ou fragilisés et/ou sensibilisés et/ou secs, et/ou améliorer la douceur et/ou la vigueur des fibres capillaires.

La présente invention vise en outre l'utilisation cosmétique, par voie orale, d'une association d'acide pétrosélinique et de zinc, en particulier des sels de Zn (II), et de préférence complexés par un ou plusieurs (poly)hydroxyacides, tel que le gluconate de zinc, pour prévenir et/ou lutter contre le vieillissement du cheveu chez l'homme de plus de 30 ans ou chez la femme.

La présente invention vise en particulier l'utilisation cosmétique, par voie orale, d'une association d'acide pétrosélinique et de zinc, en particulier des sels de Zn (II), et de préférence complexés par un ou plusieurs (poly)hydroxyacides, tel que le gluconate de zinc, pour prévenir et/ou lutter contre la microinflammation à bas bruit des follicules pileux et/ou du scalp, en particulier des follicules pileux.

Par vieillissement du cheveu on entend désigner selon l'invention un changement d'aspect de la fibre (cheveu fin, terne, sans tenue (mou), sans éclat), des cheveux qui ont tendance à chuter, qui se renouvellent moins vite, dont le réseau de collagène qui les soutient est altéré par la libération importante de collagénase et la désorganisation de son réseau, dont les parties dermique et épithéliale se rigidifient par la présence de nombreux produits de glycation (Monnier VM, 1983), dont la synthèse de sébum se réduit, entraînant une sécheresse du cuir chevelu et augmentant d'autant l'aspect terne et sans éclat du cheveu, dont l'épiderme et le derme du scalp sont aussi soumis à une désorganisation du réseau de collagène et à une importante apparition de produits de glycation, qui rigidifient le derme autour du follicule pileux entraînant des effets négatifs pour les follicules résidents et l'implantation de nouveaux cheveux.

On entend également par vieillissement du cheveu une diminution de la densité capillaire, ainsi que du diamètre des cheveux, se traduisant par une diminution de la couverture du scalp.

Par affinement des cheveux, on entend désigner selon l'invention une diminution du diamètre des cheveux en

dessous de 40 μm. Le diamètre des cheveux peut-être avantageusement mesuré à l'aide du Trichoscan, équivalent automatisé du Trichogramme, où l'oeil humain a été remplacé par un logiciel d'analyse d'image (Gasmueller, 2009). Par ailleurs, en dessous de 40 μm les cheveux sont difficilement visibles à l'oeil nu. L'affinement des cheveux pourra donc être perçu. Un seuil de 5 % pourra être considéré comme significatif.

Par pousse de cheveux épais, on entend désigner selon l'invention, la pousse de cheveux avec un diamètre supérieur à 40 μm, mesurable par le bais du Trichoscan mais aussi facilement visibles à l'oeil nu.

Par augmenter la densité capillaire, on entend selon l'invention prévenir un plus grand nombre de cheveux par cm$^2$. La densité des cheveux est également mesurable avec le Trichoscan. Avec cet appareil, une densité <280 cheveux par cm$^2$ est considérée comme une densité faible. Une augmentation de 5 % de la densité peut être considérée comme cliniquement significative et visible.

Par prévenir la chute des cheveux, on entend selon l'invention prévenir une diminution du % de cheveux en phase télogène, mesurable avec le Trichoscan, en se basant sur le fait que les cheveux en phase télogène ne poussent plus contrairement aux cheveux en phase anagène. Une diminution de 5 % du nombre de cheveux en phase télogène peut être considérée comme significative.

Par prévenir la chute des cheveux, on entend selon l'invention une diminution du % de cheveux en phase catagène, mesurable à l'aide du Trichogramme ou par recueil de cheveux au coiffage ou après la douche en se basant sur le fait que les cheveux en phase catagène sont le reflet visible des cheveux précédemment engagés en phase d'arrêt de la croissance (phase telogène) et représentent la chute telle que réellement perçue par un individu affecté par une chute anormalement élevée de cheveux. Une diminution de 5 % du nombre de cheveux en phase catagène peut être considérée comme significative.

Par augmenter le volume de la chevelure globale on entend désigner selon l'invention une augmentation du diamètre des cheveux associée à une diminution de l'hétérogénéité de leurs diamètres et à une augmentation du nombre de cheveux par cm$^2$.

Par prévenir et/ou lutter contre les cheveux mous et/ou cassants et/ou secs et/ou fragilisés et/ou ternes et/ou fourchus, on entend désigner selon l'invention une amélioration globale de la structure de la tige pilaire, et notamment de la cuticule, couche la plus externe du cheveu.

Par améliorer la douceur des cheveux, on entend désigner selon l'invention une amélioration de l'état des écailles de la cuticule et plus particulièrement de leur cohésion. Des écailles disjointes rendent les cheveux rêches au toucher.

Par améliorer la résistance à la traction des cheveux et la vigueur des fibres capillaires, on entend designer selon l'invention une amélioration de la solidité des cheveux qui peut se mesurer par le test à la traction. Cette mesure de détermination des propriétés mécaniques en traction du cheveu est réalisée à l'aide d'un outil commercial, le MTT600 (mini Tensile Tester) de la société Dia Stron. Une augmentation de 5 % de la force nécessaire pour rompre le cheveu est considérée comme significative.

Par la coiffabilité de la chevelure, on entend désigner selon l'invention un cheveu facile à peigner et/ou brosser. Les écailles disjointes s'accrochent avec les écailles des cheveux voisins provoquant l'emmêlement de la chevelure, ce qui favorise ensuite l'apparition de noeuds rendant le coiffage plus difficile.

Par coiffabilité, on entend également une meilleure tenue de la coiffure après pose de bigoudis, de brushing, de défrisage ou frisage.

Par brillance de la chevelure, on entend désigner selon la présente invention une moindre usure et/ou une réparation des écailles de la cuticule pour homogénéiser la surface du cheveu et favoriser la réflexion de la lumière.

De manière préférentielle, les différents paramètres tels que définis ci-avant pourront être évalués par des hommes et/ou des femmes au moyen d'un questionnaire à choix multiples dans le cadre d'une étude observationnelle qui pourra être réalisée chez des dermatologues. Un effet sera considéré comme significatif à partir du moment où au moins 50 % des hommes et/ou des femmes auront perçu un effet positif.

Sont particulièrement concernées par les utilisations et les procédés selon l'invention les hommes de plus de 30 ans et les femmes.

Par « homme », on entend, au sens de la présente invention, la population masculine humaine.

L'invention a également pour objet la mise en oeuvre d'une association d'actifs conforme à l'invention sous forme de complément alimentaire.

L'invention a encore pour objet une composition cosmétique destinée à une administration par voie orale ou un complément alimentaire contenant une association d'acide pétrosélinique, de zinc, en particulier un sel de Zn (II), et de préférence complexé par un ou plusieurs (poly)hydroxyacides, tel que le gluconate de zinc, et de taurine, dans laquelle l'acide pétrosélinique est présent dans une teneur comprise entre 20 et 70 % en poids par rapport au poids total de ladite association d'actifs.

De préférence, une telle composition ou un tel complément alimentaire comprend en outre de la vitamine D3 et de l'acétate de tocophérol.

Par zinc, on entend le zinc ou l'un de ses sels (acétate, chlorure, citrate, lactate, gluconate, lactate, oxyde, carbonate ou sulfate de zinc), en particulier les sels de Zn (II), et de préférence compléxés par un ou plusieurs (poly)hydroxyacides, tel que le gluconate de zinc.

Par (poly)hydroxyacide, on entend tout acide carboxylique qui comprend une chaine hydrocarbonée, linéaire ou ramifiée, saturée ou insaturée, de préférence saturée et/ou linéaire, comprenant de 1 à 10 atomes de carbone et de 1 à 9 groupes hydroxy, et comprenant de 1 à 4 groupes carboxyliques -C(O)-OH, dont au moins une desdites fonctions -C(O)-OH est sous la forme carboxylate -C(O)-O- complexée avec l'atome de Zn, de préférence Zn(II).

Plus particulièrement le sel de zinc est complexe par deux groupes carboxylates tels que celui de formule (I) :

$$R\text{-}C(O)\text{-}O\text{-}Zn\text{-}O\text{-}C(O)\text{-}R' \qquad (I)$$

dans laquelle R et R', identiques ou différents, représentent un groupe (C1 C6)(poly)hydroxyalkyle, ainsi que ses solvates tels que les hydrates et leurs énantiomères.

De préférence le composé de formule (I) est du gluconate de zinc.

Selon un mode de réalisation particulier de l'invention, le zinc n'est pas un oxyde de zinc mais un sel de zinc. Par Zn(II), on entend un atome de zinc de degré d'oxydation $Zn^{2+}$.

Dans la mesure où le produit selon l'invention est destinée à une mise en oeuvre par voie orale chez un individu, les sels pouvant être mis en oeuvre sont bien évidemment choisis pour leur totale innocuité.

L'invention concerne également un procédé cosmétique pour prévenir et/ou lutter contre la microinflammation à bas bruit des follicules pileux et/ou du scalp, permettant d'(de):

- améliorer la qualité de la chevelure ; et/ou
- prévenir et/ou lutter contre le vieillissement du cheveu chez l'homme de plus de 30 ans ou chez la femme ,

comprenant au moins l'administration, par voie orale, à un individu, d'une association d'actifs conforme à l'invention, ou d'une composition orale ou d'un complément alimentaire comprenant une telle association d'actifs.

Les procédés selon l'invention présentent les caractéristiques de procédés cosmétiques dans la mesure notamment où ils permettent d'améliorer l'esthétique de la chevelure, en particulier en luttant contre l'affinement progressif des cheveux apparaissant notamment avec l'âge. En outre, une association d'actifs, une composition ou un complément alimentaire selon l'invention peut être utilisé quotidiennement pendant plusieurs mois, sans prescription médicale. La présente invention se situe donc clairement en dehors du champ thérapeutique.

En particulier, les utilisations et/ou procédés cosmétiques de l'invention permettent de protéger les cheveux vieillis, notamment chez l'homme de plus de 30 ans ou chez la femme.

L'invention concerne également un complément alimentaire comprenant une partie des composés formant l'association d'actifs conforme à l'invention dans une première composition, et au moins l'autre partie des composés formant l'association d'actifs dans une deuxième composition, comme kit ou produit de combinaison pour une utilisation simultanée, séparée ou étalée dans le temps.

Il est entendu dans le cadre de la présente invention que « l'utilisation cosmétique par voie orale » recouvre l'utilisation de produits administrés par voie orale, ces produits étant par exemple sous forme de complément alimentaire comme exposé ci-après. Ces produits produisent un effet, au niveau de la chevelure, sur le plan esthétique et du confort, ou encore à visée beauté, par exemple en vue de la protéger, de la maintenir en bon état, et notamment de l'embellir, notamment par une augmentation du volume et de la tenue de la chevelure globale.

On entend par huile riche en acide pétrosélinique une huile comprenant au moins 20 % d'acide pétrosélinique et plus préférentiellement plus de 30 % d'acide pétrosélinique.

Alternativement, l'acide pétrosélinique, ou acide gras mono-insaturé (C18 :1 n-12 ou cis delta 6) ou acide delta-6-cis-octadecenoique en C18, est mis en oeuvre dans une association d'actifs conforme à l'invention.

Les ombellifères sont des plantes dont les fleurs sont disposées en ombelles. Les espèces particulièrement riches en acide pétrosélinique sont les Umbellifarea-Apiacea et Araliaceae. Les plantes du genre Thapsia sont également des sources d'acide pétrosélinique (Avato et al, Lipids, 2001, 36, 845). Les espèces de préférence utilisées dans l'invention sont le coriandre, le cerfeuil, la carotte, le céleri, le cumin, le carvi, le persil et l'aneth. L'huile d'ombellifère utilisée selon l'invention peut être extraite des graines de ces ombellifères, par exemple par broyage ou pressage, puis raffinage. L'huile d'ombellifère a une teneur en acide pétrosélinique qui varie selon la graine d'ombellifère dont elle est extraite. Pour une même ombellifère, la teneur en acide pétrosélinique varie aussi selon le pays d'origine de l'ombellifère et selon l'extraction qui peut être plus ou moins complète.

L'acide pétrosélinique est également un composé abondant (environ 48 %) de l'huile de graine de *Geranium sanguineum,* ainsi que de l'huile de graines de coriandre *(Coriandrum sativum).*

Ainsi, selon un mode de réalisation, l'utilisation objet de la présente invention est telle que l'acide pétrosélinique est utilisé sous forme d'huile d'ombellifère ou de *Geranium sanguineum,* de préférence sous la forme d'une huile de coriandre *(Coriandrum sativum)*, de cerfeuil, de carotte, de céleri, de cumin, de carvi, de persil ou d'aneth, préférentiellement sous forme d'une huile de graines de coriandre (*Coriandrum sativum*).

Les teneurs en acide pétrosélinique sont variables selon que l'association d'actifs conformes à l'invention est mise en oeuvre dans une composition cosmétique destinée à une administration par voie orale ou dans un complément alimentaire.

Des teneurs sont indiquées ci-après à titre indicatif.

La teneur en acide pétrosélinique, dans une composition cosmétique destinée à l'administration par voie orale ou dans un complément alimentaire conforme à l'invention, peut être comprise entre 1 et 70 % du poids total, notamment entre 10 et 70 % du poids total, particulièrement entre 15 et 70 % du poids total de la composition ou du complément. La teneur en acide pétrosélinique dans une composition cosmétique destinée à l'administration par voie orale ou dans un complément alimentaire conformes l'invention, pour la voie orale, peut être telle que la dose journalière dudit acide pétrosélinique est comprise entre 5 et 1000 mg/j, et notamment entre 50 et 650 mg/j.

Selon une variante privilégiée, la composition cosmétique ou un complément alimentaire conformes à l'invention comprend en outre de la vitamine D3.

Selon une variante privilégiée, la composition cosmétique ou un complément alimentaire conformes à l'invention comprend en outre de l'acétate de tocophérol.

Selon une variante privilégiée, une composition cosmétique ou un complément alimentaire conformes à l'invention comprend en outre au moins un acide aminé choisi parmi les acides aminés constitutifs et/ou non constitutifs des protéines, en particulier l'arginine, la cystéine et/ou la méthionine.

Par taurine selon présente invention, on entend la taurine, l'hypotaurine ou l'un de leurs sels. Dans la mesure où l'association selon l'invention est destinée à une mise en oeuvre par voie orale chez un individu, les sels pouvant être mis en oeuvre sont bien évidemment choisis pour leur totale innocuité. Conviennent à ce titre les sels alcalins ou alcalinoterreux, en particulier les sels de magnésium, manganèse, fer II ou zinc.

La teneur en taurine, hypotaurine ou l'un de ses sels, dans une composition cosmétique destinée à l'administration par voie orale ou dans un complément alimentaire conforme à l'invention, peut être comprise entre 1 et 40 % du poids total de la composition, notamment entre 5 et 40 % du poids total de la composition, particulièrement entre 5 et 30 % du poids total de la composition ou du complément.

La teneur en taurine, hypotaurine ou l'un de ses sels, dans une composition cosmétique destinée à l'administration par voie orale ou complément alimentaire conforme à l'invention, pour la voie orale, peut être telle que la dose journalière de ladite taurine, hypotaurine ou l'un de ses sels est comprise entre 4 et 700 mg/j, et notamment entre 40 et 300 mg/j.

La teneur en zinc, en particulier en gluconate de zinc, dans une composition cosmétique destinée à l'administration par voie orale ou complément alimentaire conforme à l'invention, peut être comprise entre 0,001 % et 30 % en poids, notamment entre 0,01 et 25 % en poids, particulièrement entre 0,1 et 20 % en poids, par rapport au poids total de l'association d'actifs.

La teneur en gluconate de zinc, dans une composition cosmétique destinée à l'administration par voie orale ou dans un complément alimentaire conformes à l'invention, peut être telle que la dose journalière dudit gluconate de zinc est comprise entre 0,01 et 300 mg/j, notamment entre 0,1 et 200 mg/j, particulièrement entre 1 et 100 mg/j.

Selon un mode de réalisation, une composition cosmétique destinée à l'administration par voie orale ou un complément alimentaire conformes à l'invention peut en outre comprendre au moins une vitamine choisie parmi la vitamine B1, B5, B6, B8, B9, B12, C, D, et notamment D3, PP, ou le tocophérol (vitamine E) et ses dérivés, notamment ester tel que l'acétate, le succinate ou le palmitate de tocophérol, de préférence de l'acétate de tocophérol.

Selon ce mode de réalisation, une composition cosmétique destinée à une administration par voie orale ou un complément alimentaire conforme à la présente invention tel(le) que décrit plus haut comprend de préférence au moins de la vitamine E ou l'un de ses dérivés et/ou de la vitamine D, préférentiellement de la vitamine D3 et/ou de l'acétate de tocophérol.

Selon un mode de réalisation particulier, une composition cosmétique destinée à une administration par voie orale ou un complément alimentaire conformes à la présente invention comprend en outre de la vitamine D3 et de l'acétate de tocophérol.

Ainsi, selon un mode de réalisation préféré de l'invention, la présente invention vise une composition cosmétique destinée à une administration par voie orale ou un complément alimentaire comprenant de l'acide pétrosélinique, du zinc et en particulier un sel de Zn (II), et de préférence complexé par un ou plusieurs (poly)hydroxyacides, de préférence du gluconate de zinc, de la taurine, de la vitamine D3 et de l'acétate de tocophérol, dans laquelle l'acide pétrosélinique est présent dans une teneur comprise entre 20 et 70% en poids par rapport au poids total de ladite association d'actifs

Les compositions selon l'invention peuvent en outre comprendre au moins un caroténoïde, notamment un caroténoïde choisi parmi le β-carotène, le lycopène, l'astaxanthine, la zéaxanthine et la lutéine, des flavonoïdes tels que les les anthocyanes, les flavonols, les flavanols (proanthocyanidines des extraits de raisin, catéchines des extraits de thé vert), les flavanones (hespéridine, diosmin), les acides phénoliques et dérivés (acide chlorogénique des extraits de café), les diterpènes, les stilbènes, les extraits de chicorés, des extraits de ginkgo biloba, des extraits de piment, des extraits de soja, d'autres sources de flavonoïdes possédant des propriétés antioxydantes, des acides gras, des prébiotiques, des probiotiques, du resveratrol, des acides aminés, du sélénium et des précurseurs de glutathion.

Les compositions orales ou les compléments alimentaires conformes à l'invention peuvent en outre comprendre au moins un probiotique, un prébiotique ou un mélange de probiotiques et un mélange de prébiotiques. A titre de microorganismes probiotiques, on peut notamment citer *Lactobacillus johnsonii* ou *Lactobacillus paracaseï.*

Les compositions cosmétiques destinées à une administration par voie orale ou compléments alimentaires conformes à la présente invention peuvent se présenter sous toutes les formes galéniques normalement utilisées pour la voie orale.

Dans le présent texte, lorsqu'il est discuté des bornes x à y, on entend les bornes x et y incluses.

Complément alimentaire

Selon un mode de réalisation, une composition cosmétique destinée à une administration par voie orale ou un complément alimentaire conforme à l'invention comprend :

(i) de l'acide pétrosélinique en une teneur comprise entre 20 et 70 % en poids, par rapport au poids total de l'association d'actifs ;

(ii) au moins un (poly)hydroxyacide de zinc, de préférence du gluconate de zinc, en une teneur comprise entre 0,001 et 40 % en poids, notamment entre 0,01 et 25 % en poids, particulièrement entre 0,1 et 20 % en poids, par rapport au poids total de l'association d'actifs ;

(iii) de la taurine en une teneur comprise entre 1 et 50 % en poids, notamment entre 5 et 40 % en poids, particulièrement entre 10 et 30 % en poids, par rapport au poids total de l'association d'actifs ; et/ou

(iv) optionnellement de la vitamine D3 en une teneur comprise entre 0,0001 et 1,0 % en poids, notamment entre 0,0001 et 0,5 % en poids, particulièrement entre 0,0001 et 0,1 % en poids, par rapport au poids total de l'association d'actifs ; et

(v) optionnellement de l'acétate de tocophérol en une teneur comprise entre 0,01 et 10 % en poids, notamment entre 0,1 et 10 % en poids, particulièrement entre 0,2 et 5 % en poids, par rapport au poids total de l'association d'actifs.

Selon un mode de réalisation particulier, une composition cosmétique destinée à une administration par voie orale ou un complément alimentaire conformes à l'invention comprend les ingrédients i) à v) suivants, pris ensembles ou indépendamment :

(i) de l'acide pétrosélinique en une teneur comprise entre 1 et 70 % en poids, notamment entre 10 et 70 % en poids, particulièrement entre 15 et 70 % en poids, par rapport au poids total de la composition ou du complément ;

(ii) au moins un (poly)hydroxyacide de zinc, de préférence du gluconate de zinc, en une teneur comprise entre 0,001 et 30 % en poids, notamment entre 0,01 et 25 % en poids, particulièrement entre 0,1 et 20 % en poids, par rapport au poids total de la composition ou du complément ;

(iii) de la taurine en une teneur comprise entre 1 et 40 % en poids, notamment entre 5 et 40 % en poids, particulièrement entre 5 et 30 % en poids, par rapport au poids total de la composition ou du complément ; et/ou

(iv) optionnellement de la vitamine D3 en une teneur comprise entre 0,0001 et 1,0 % en poids, notamment entre 0,0001 et 0,5 % en poids, particulièrement entre 0,0001 et 0,1 % en poids, par rapport au poids total de la composition ou du complément ; et/ou

(v) optionnellement de l'acétate de tocophérol en une teneur comprise entre 0,01 et 10 % en poids, notamment entre 0,1 et 10 % en poids, particulièrement entre 0,2 et 5 % en poids, par rapport au poids total de la composition ou du complément.

Selon un mode de réalisation particulier, la composition cosmétique destinée à une administration par voie orale ou le complément alimentaire conformes à l'invention comprend l'ensemble des ingrédients (i) à (v) susmentionnés.

Une telle composition de type complément alimentaire ou une composition orale conforme à l'invention peut en particulier présenter les teneurs suivantes :

| Composants | % en poids par rapport au poids total de la composition |
|---|---|
| Acide pétrosélinique | 54,9 *(apporté par l'huile de graines de coriandre)* |

(suite)

| Composants | % en poids par rapport au poids total de la composition |
|---|---|
| Gluconate de zinc | 6,3 (dont 13,6 % de matière active) |
| Taurine | 18,7 (dont 98,5 % de matière active) |
| Vitamine E | 1,0 (dont 67 % de matière active) |
| Vitamine D3 | 0,03 (dont 2,5 % de matière active) |

Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, tablettes ou capsules molles.

Les compositions selon l'invention, destinées à une administration par voie orale, peuvent notamment comprendre l'intégralité ou une partie seulement de la dose journalière.

Autrement dit, une à trois compositions peuvent être administrées par jour.

Typiquement, la durée de ce traitement cosmétique destinée à une administration par voie orale peut être supérieure à 4 semaines, notamment de 4 à 24 semaines, avec le cas échéant une ou plusieurs périodes d'interruption.

Le complément alimentaire conforme à la présente invention peut comprendre une partie des actifs formant l'association selon l'invention dans une première composition et l'autre partie de ces actifs dans une deuxième composition, comme kit ou produit de combinaison pour une utilisation simultanée, séparée ou étalée dans le temps.

Ce complément peut être formulé de telle façon que les deux compositions sont sous les mêmes formes ou sous des formes différentes, par exemple choisies parmi celles citées plus haut. Un tel kit peut notamment être présenté dans un seul et même emballage.

Bien entendu l'homme du métier veillera à choisir les éventuels additifs complémentaires et/ou leur quantité de telle manière que les propriétés avantageuses de l'association selon l'invention ou de la composition comprenant l'association selon l'invention ne soient pas ou substantiellement pas altérées par l'adjonction envisagée.

Les actifs selon l'invention peuvent être formulés avec les excipients et composants usuels pour des compositions orales ou des compléments selon l'invention, à savoir notamment des composants gras et/ou aqueux, des agents humectants, des épaississants, des conservateurs, des agents de texture et/ou d'enrobage, des antioxydants, les arômes et des colorants usuels dans le domaine des complément alimentaire.

Comme actif cosmétique autre que l'association d'actifs conforme à l'invention, une composition ou un complément alimentaire conformes à l'invention peut contenir un actif additionnel hydrophile choisi parmi les sucres et les dérivés de sucre, les vitamines hydrosolubles, les extraits végétaux par exemple de romarin, d'écorce de pin ou de fruits comme l'orange, les peptides et acides aminés comme l'arginine, la méthionine, la cystéine, la citrulline et/ou un actif additionnel lipophile choisi parmi le rétinol (vitamine A) et ses précurseurs, notamment le Beta carotène, les caroténoïdes antioxydants comme le lycopène, la zeaxanthine, l'astaxanthine et la lutéine, les acides gras essentiels, les huiles végétales et animales notamment les huiles de poisson riches en oméga 3 essentielles, les phospholipides comme la lécithine, et leurs mélanges, les huiles de cucurbitacés comme la courge ou les sources d'enzymes antioxidantes comme le melon, les probiotiques et les prébiotiques, les vitamines et minéraux, les acides gras polyinsaturés et mono insaturées PUFA et MUFA et les oméga 3 et oméga 6.

D'autres caractéristiques et avantages de l'invention ressortiront mieux des exemples qui suivent, donnés à titre illustratif et non limitatif.

**Exemple 1**

**Composition pour la voie orale sous forme de capsule molle.**

| Ingrédients | (mg/capsule molle) |
|---|---|
| Huile de graines de coriandre (65% acide pétrosélinique) | 300 |
| Taurine | 76,10 |
| Gluconate de zinc | 25,75 |
| Vitamine E | 4,10 |

(suite)

| Ingrédients | (mg/capsule molle) |
|---|---|
| Vitamine D3 | 0,115 |
| **Excipients** | |
| Huile de coco, raffinée | 112 |
| Cire d'abeille, jaune, Cera flava | 22 |
| Sunflower lecithin | 10 |
| **Capsule** | |
| Gélatine de poisson | 144,6 |
| Glycérol | 58,6 |
| Eau purifiée | 6,8 |

**Exemple 2**

Composition pour la voie orale sous forme de capsule molle.

| Ingrédients | (mg/capsule molle) |
|---|---|
| Huile de graines de coriandre (65% acide pétrosélinique) | 300 |
| Gluconate de zinc | 25,75 |
| Vitamine E | 4,10 |
| **Excipients** | |
| Huile de coco, raffinée | 112 |
| Cire d'abeille, jaune Cera flava | 22 |
| Lécithine de tournesol | 10 |
| **Capsule** | |
| Gélatine de poisson | 144,6 |
| Glycérol | 58,6 |
| Eau purifiée | 6,8 |

**Exemple 3**

Compositions pour la voie orale sous forme d'émulsion en stick.

A.

| Ingrédients | (g/stick) |
|---|---|
| Huile de graines de coriandre (dont 65% d'acide pétrosélinique) | 0,40 |
| Gluconate de zinc | 0,051 |
| Vitamine E | 0,0082 |
| **Excipients** | |
| Eau | 1,722 |

(suite)

| Excipients | |
|---|---|
| Sucre | 0,911 |
| Fructose | 0,911 |
| Microcristalline cellulose | 0,032 |
| Carboxymethyl cellulose sodique | 0,004 |
| Mélange naturel de tocophérols | 0,034 |
| Huile de tournesol | 1,015 |
| Arôme naturel de citron | 0,034 |
| Sorbate de potassium | 0,013 |
| Acide citrique | 0,013 |
| Alginate de propylène glycol | 0,01 |

**B.**

| Ingrédients | (g/stick) |
|---|---|
| Huile de graines de coriandre (dont 65% d'acide pétrosélinique) | 0,40 |
| Sulfonate de Zinc | 0,051 |
| Vitamine E | 0,0082 |
| **Excipients** | |
| Eau | 1,722 |
| Sucre | 0,911 |
| Fructose | 0,911 |
| Microcristalline cellulose | 0,032 |
| Carboxymethyl cellulose sodique | 0,004 |
| Mélange naturel de tocophérols | 0,034 |
| Huile de tournesol | 1,015 |
| Arôme naturel de citron | 0,034 |
| Sorbate de potassium | 0,013 |
| Acide citrique | 0,013 |
| Alginate de propylène glycol | 0,01 |

### Exemple 4

**Mise en évidence de l'effet d'une association d'acide pétrosélinique et de gluconate de zinc sur la production d'une résolvine anti-inflammatoire endogène issue du métabolisme lipidique: la lipoxine A4 dans un modèle expérimental utilisant des cellules mononuclées humaines in vitro.**

Des cellules mononuclées sanguines sont mises en culture sous 5 % de $CO_2$ et à 37°C dans un milieu sans sérum pour macrophages (SFM Macrophage ; Invitrogen 12065074) pendant 24 heures. A l'issue de cette étape, le milieu est remplacé par le même milieu d'essai frais contenant en plus les actifs aux différentes doses pendant 30 minutes en présence des différents produits à évaluer (Huile de coriandre 0,25 mg/ml, Gluconate de Zinc (0,005 mg/ml) La réponse inflammatoire a ensuite été déclenchée en présence de phorbol myristate (0,05 $\mu$M) et du calcium ionophore (1 $\mu$M) et d'un mélange de substrat lipidique composé d'acide docosahexaénoïque (DHA - 1 $\mu$g/mL) et d'acide eicosapentaénoïque (EPA - 1 µg/mL).

Les surnageants ont ensuite été prélevés au bout de 2 h de stimulation et congelés à -80°C avant préparation pour analyse en spectrométrie de masse.

Des triplicats expérimentaux (trois puits) ont été effectués par condition expérimentale. Dans chaque plaque de culture a été introduit un contrôle correspondant à des cellules stimulées par le mélange PMA/A23187 et/ou avec ajout du mélange équimolaire d'acides gras.

Les surnageants décongelés ont été concentrés par extraction en phase solide (SPE), repris dans du méthanol avant analyse spectométrique. La méthode analytique utilisée consiste à séparer les différents analytes par chromatographie liquide haute pression en fonction de leur temps de rétention et de les quantifier par spectrométrie de masse.

Les analyses ont été effectuées sur une chaine LC 1290 Infinity (Agilent Technologies) couplée à un spectromètre de masse 6460 Triple Quad LC/MS (Agilent Technologies) équipé d'une source d'ionisation en electrospray (Jet stream technology) opérant en monde négatif. Les séparations chromatographiques ont été réalisées sur une colonne ZorBAX SB-C18.

Les résultats ont été obtenus en pg/mL de surnageant cellulaire. Ces données brutes ont été ensuite transformées par calcul pour obtenir le pourcentage d'activation (ou d'inhibition) par rapport au témoin de la plaque en utilisant le calcul suivant :

$$\% \text{ de modulation} = 100 \text{ x (valeur obtenue avec l'actif} - \text{valeur du témoin)/valeur du témoin}$$

Ces pourcentages de modulation sont reportés dans le tableau ci-après.

Une association d'actifs conforme à l'invention comprenant de l'huile de coriandre, riche en acide pétrosélinique, et du gluconate de zinc, ainsi que ces mêmes composés de façon individuelle, ont été testées conformément à ce qui est indiqué ci-dessus.

Les résultats obtenus à l'issu de ces tests comparatifs sont les suivants :

| Composés testés | Niveau de production de la lipoxine A4 |
|---|---|
| Huile de coriandre *(dont entre 60 et 75 % d'acide pétrosélinique)* 0.25 mg/ml | +19 % |
| Gluconate de zinc 0.005 mg/ml | -5 % |
| Gluconate de zinc 0.005mg/ml + Huile de coriandre *(dont entre 60 et 75 % d'acide pétrosélinique )* 0.25 mg/ml | **+94 %** |

Dans ce cas également, on peut voir que l'effet d'une association conforme à l'invention sur la production de lipoxine A4 est très nettement supérieur à la somme des effets des composés mis en oeuvre individuellement.

En effet, on a pu observer une augmentation de la production de lipoxine A4 de 94 % par rapport au niveau basal de production de ce composant anti-inflammatoire lorsque les cellules lymphocytaires testées ont été mises en contact avec l'association d'actifs.

C'est donc bien un effet synergique d'une association d'actifs conforme à l'invention qui est ici observé et démontré.

**Revendications**

1. Utilisation cosmétique, par voie orale, d'une association d'acide pétrosélinique et de zinc, en particulier les sels de Zn (II), et de préférence complexés par un ou plusieurs (poly)hydroxyacides, tel que le gluconate de zinc, pour prévenir et/ou lutter contre la microinflammation à bas bruit des follicules pileux, permettant d'améliorer la qualité de la fibre capillaire, en particulier chez l'homme de plus de 30 ans ou chez la femme, **caractérisée en ce que** l'amélioration de la qualité de la fibre capillaire comprend améliorer la résistance à la traction des cheveux, et/ou au coiffage et/ou à la mise en forme des cheveux et/ou prévenir et/ou lutter contre les cheveux mous et/ou cassants et/ou ternes et/ou fourchus et/ou fragilisés et/ou sensibilisés et/ou secs, et/ou améliorer la douceur et/ou la vigueur des fibres capillaires.

2. Utilisation selon la revendication 1, **caractérisée en ce que** l'acide pétrosélinique est utilisé sous forme d'huile d'ombellifère ou de *Geranium sanguineum,* de préférence sous la forme d'une huile de coriandre, de cerfeuil, de carotte, de céleri, de cumin, de carvi, de persil ou d'aneth, préférentiellement sous la forme d'une huile de graines de coriandre.

3. Utilisation selon l'une quelconque des revendications précédentes, **caractérisée en ce que** de la taurine est utilisée par voie orale, conjointement à ladite association.

4. Utilisation selon l'une quelconque des revendications précédentes, **caractérisée en ce que** de la vitamine D3 est utilisée par voie orale, conjointement à ladite association.

5. Utilisation selon l'une quelconque des revendications précédentes, **caractérisée** en ce queles actifs sont mis en oeuvre sous forme d'un complément alimentaire.

6. Utilisation selon la revendication 5, dans laquelle le complément alimentaire comprend en outre au moins une vitamine choisie parmi la vitamine B1, B5, B6, B8, B9, B12, C, D, de préférence D3, PP, ou la vitamine E et ses dérivés, et comprend de préférence de la vitamine D et/ou de la vitamine E ou l'un de ses dérivés, préférentiellement de la vitamine D3 et/ou de l'acétate de tocophérol, plus préférentiellement de la vitamine D3 et de l'acétate de tocophérol.

7. Utilisation selon l'une quelconque des revendications 5 ou 6, dans laquelle le complément alimentaire comprend en outre au moins un acide aminé choisi parmi les acides aminés constitutifs et/ou non constitutifs des protéines, en particulier l'arginine, la cystéine et/ou la méthionine.

8. Utilisation selon l'une quelconque des revendications 5 à 7, dans laquelle le complément alimentaire comprend une association d'acide pétrosélinique, de taurine, et de zinc, et en particulier de sels de Zn (II), et de préférence compléxés par un ou plusieurs (poly)hydroxyacides, tel que le gluconate de zinc.

9. Utilisation selon l'une quelconque des revendications 5 à 8, dans laquelle le complément alimentaire comprend de l'acide pétrosélinique, du zinc et en particulier un sel de Zn (II), et de préférence complexé par un ou plusieurs (poly)hydroxyacides, de préférence le gluconate de zinc, de la taurine, de la vitamine D3 et de l'acétate de tocophérol.

10. Complément alimentaire, contenant une association d'acide pétrosélinique, de zinc, en particulier un sel de Zn (II), et de préférence complexé par un ou plusieurs (poly)hydroxyacides, tel que le gluconate de zinc, et de taurine, dans laquelle l'acide pétrosélinique est présent dans une teneur comprise entre 20 et 70 % en poids par rapport au poids total de ladite association d'actifs.

11. Complément alimentaire selon la revendication précédente, comprenant en outre de la vitamine D3 et de l'acétate de tocophérol.

12. Complément alimentaire selon la revendication 10 ou 11, comprenant :

(i) de l'acide pétrosélinique en une teneur comprise entre 1 et 70 % en poids, notamment entre 10 et 70 % en poids, particulièrement entre 15 et 70 % en poids, par rapport au poids total du complément ;
(ii) au moins un (poly)hydroxyacide de zinc, de préférence du gluconate de zinc en une teneur comprise entre 0,001 et 30 % en poids, notamment entre 0,01 et 25 % en poids, particulièrement entre 0,1 et 20 % en poids, par rapport au poids total du complément ;
(iii) de la taurine en une teneur comprise entre 1 et 40 % en poids, notamment entre 5 et 40 % en poids, particulièrement entre 5 et 30 % en poids, par rapport au poids total du complément ; et/ou
(iv) optionnellement de la vitamine D3 en une teneur comprise entre 0,0001 et 1 % en poids, notamment entre 0,0001 % et 0,5 % en poids, particulièrement entre 0,0001 et 0,1 % en poids, par rapport au poids total du complément ; et/ou
(v) optionnellement de l'acétate de tocophérol en une teneur comprise entre 0,01 et 10 % en poids, notamment entre 0,1 et 10 % en poids, particulièrement entre 0,2 et 5 % en poids, par rapport au poids total du complément.

13. Procédé cosmétique pour améliorer la qualité de la chevelure, en particulier chez l'homme de plus de 30 ans ou chez la femme, comprenant au moins l'administration, par voie orale, à un individu, des actifs tels que définis dans l'une quelconque des revendications 1 à 9 ou d'un complément alimentaire tel que défini dans l'une quelconque des revendications 10 à 12.

**EP 2 994 091 B1**

14. Complément alimentaire comprenant une partie des actifs tels que définis en revendications 1 à 9 dans une première composition, et au moins l'autre partie des actifs dans une deuxième composition, comme kit ou produit de combinaison pour une utilisation simultanée, séparée ou étalée dans le temps.

**Patentansprüche**

1. Kosmetische, orale, Verwendung einer Kombination aus Petroselinsäure und Zink, insbesondere Salze von Zn(II), und vorzugsweise komplexiert mit einer oder mehreren (Poly)hydroxysäuren, wie Zinkgluconat, zur Vorbeugung und/oder Bekämpfung der schleichenden Mikroinflammination von Haarfollikeln, die die Verbesserung der Qualität der Haarfaser, insbesondere bei Männern über 30 Jahren oder bei Frauen ermöglicht, **dadurch gekennzeichnet, dass** die Verbesserung der Qualität der Haarfaser die Verbesserung der Widerstandsfähigkeit gegenüber Ziehen des Haars und/oder Frisieren und/oder Umformen des Haars und/oder die Verhinderung und/oder Bekämpfung von kraftlosem und/oder brüchigem und/oder glanzlosem und/oder gespaltenem und/oder brüchigem und/oder empfindlichem und/oder trockenem Haar und/oder die Verbesserung der Geschmeidigkeit und/oder Kräftigkeit der Haarfasern umfasst.

2. Verwendung nach Anspruch 1, **dadurch gekennzeichnet, dass** Petroselinsäure in Form von Doldenblütleröl oder Öl von *Geranium sanguineum,* vorzugsweise in Form von Koriander-, Kerbel-, Karotten-, Sellerie-, Kreuzkümmel-, Kümmel-, Petersilie- oder Dillöl, vorzugsweise in Form von einem Öl aus Koriandersamen verwendet wird.

3. Verwendung nach einem der vorangehenden Ansprüche, **dadurch gekennzeichnet, dass** Taurin oral, zusammen mit der Kombination verwendet wird.

4. Verwendung nach einem der vorangehenden Ansprüche, **dadurch gekennzeichnet, dass** Vitamin D3 oral, zusammen mit der Kombination verwendet wird.

5. Verwendung nach einem der vorangehenden Ansprüche, **dadurch gekennzeichnet, dass** die Wirkstoffe als Nahrungsergänzungsmittel verwendet werden.

6. Verwendung nach Anspruch 5, wobei das Nahrungsergänzungsmittel ferner mindestens ein Vitamin umfasst, ausgewählt aus Vitamin B1, B5, B6, B8, B9, B12, C, D, vorzugsweise D3, PP oder Vitamin E und seinen Derivaten, und vorzugsweise Vitamin D und/oder Vitamin E oder einem seiner Derivate, vorzugsweise Vitamin D3 und/oder Tocopherolacetat, stärker bevorzugt Vitamin D3 und Tocopherolacetat.

7. Verwendung nach einem der Ansprüche 5 oder 6, wobei das Nahrungsergänzungsmittel ferner mindestens eine Aminosäure umfasst, ausgewählt aus Proteine konstituierenden und/oder nicht-konstituierenden Aminosäuren, insbesondere Arginin, Cystein und/oder Methionin.

8. Verwendung nach einem der Ansprüche 5 bis 7, wobei das Nahrungsergänzungsmittel eine Kombination aus Petroselinsäure, Taurin und Zink, insbesondere Salze von Zn(II), und vorzugsweise komplexiert mit einer oder mehreren (Poly)hydroxysäuren, wie Zinkgluconat, umfasst.

9. Verwendung nach einem der Ansprüche 5 bis 8, wobei das Nahrungsergänzungsmittel Petroselinsäure, Zink und insbesondere ein Salz von Zn(II) umfasst und vorzugsweise mit einer oder mehreren (Poly)hydroxysäuren, vorzugsweise Zinkgluconat, Taurin, Vitamin D3 und Tocopherolacetat, komplexiert ist.

10. Nahrungsergänzungsmittel, enthaltend eine Kombination aus Petroselinsäure, Zink, insbesondere ein Salz von Zn(II), und vorzugsweise komplexiert mit einer oder mehreren (Poly)hydroxysäuren, wie Zinkgluconat, und Taurin, wobei die Petroselinsäure in einem Gehalt von zwischen 20 und 70 Gew.-%, bezogen auf das Gesamtgewicht der Kombination von Wirkstoffen, vorhanden ist.

11. Nahrungsergänzungsmittel nach dem vorangehenden Anspruch, ferner umfassend Vitamin D3 und Tocopherolacetat.

12. Nahrungsergänzungsmittel nach Anspruch 10 oder 11, umfassend:

i) Petroselinsäure in einem Gehalt von 1 bis 70 Gew.-%, insbesondere von 10 bis 70 Gew.-%, insbesondere von 15 bis 70 Gew.-%, bezogen auf das Gesamtgewicht des Ergänzungsmittels;

(ii) mindestens eine Zink-(Poly)hydroxysäure, vorzugsweise Zinkgluconat, mit einem Gehalt von 0,001 bis 30 Gew.-%, insbesondere von 0,01 bis 25 Gew.-%, insbesondere von 0,1 bis 20 Gew.-%, bezogen auf das Gesamtgewicht des Ergänzungsmittels;

iii) Taurin in einem Gehalt von 1 bis 40 Gew.-%, insbesondere 5 bis 40 Gew.-%, insbesondere 5 bis 30 Gew.-%, bezogen auf das Gesamtgewicht des Ergänzungsmittels; und/oder

iv) gegebenenfalls Vitamin D3 in einem Gehalt von 0,0001 bis 1 Gew.-%, insbesondere von 0,0001 bis 0,5 Gew.-%, insbesondere von 0,0001 bis 0,1 Gew.-%, bezogen auf das Gesamtgewicht des Ergänzungsmittels; und/oder

(v) gegebenenfalls Tocopherolacetat in einem Gehalt von 0,01 bis 10 Gew.-%, insbesondere von 0,1 bis 10 Gew.-%, insbesondere von 0,2 bis 5 Gew.-%, bezogen auf das Gesamtgewicht des Ergänzungsmittels.

13. Kosmetisches Verfahren zur Vorbeugung und/oder Bekämpfung von schleichender Mikroinflammation der Haarfollikeln und/oder Kopfhaut, wodurch sich die Haarqualität verbessern lässt, insbesondere bei Männern im Alter von über 30 Jahren oder bei Frauen, umfassend mindestens die orale Verabreichung von Wirkstoffen an eine Person nach einem der Ansprüche 1 bis 9 oder einem Nahrungsergänzungsmittel nach einem der Ansprüche 10 bis 12.

14. Nahrungsergänzungsmittel, umfassend einen Teil der Wirkstoffe nach den Ansprüchen 1 bis 9 in einer ersten Zusammensetzung und mindestens den anderen Teil der Wirkstoffe in einer zweiten Zusammensetzung als Kit oder Kombinationsprodukt zur gleichzeitigen, getrennten oder zeitlich verzögerten Verwendung.


**Claims**

1. Cosmetic use, via oral route, of an association of petroselinic acid and zinc, in particular Zn (II) salts, and preferably complexed with one or more polyhydroxy acids such as zinc gluconate, to prevent and/or combat low-noise microinflammation of hair follicles, allowing an improvement in the quality of hair fibre, in particular in men over the age of 30 years and in women, **characterized in that** the improvement in hair fibre quality comprises an improvement in the tensile strength of hair and/or resistance to hair styling and/or hair shaping and/or to prevent and/or combat limp and/or brittle and/or dull hair and/or split ends and/or fragile and/or sensitized and/or dry hair, and/or to improve the softness and/or vigour of hair fibres.

2. The use according to claim 1, **characterized in that** the petroselinic acid is used in the form of an umbellifer plant oil or *Geranium sanguineum* oil, preferably in the form of coriander, chervil, carrot, celery, cumin, caraway, parsley or dill oil, preferably in the form of coriander seed oil.

3. The use according to any of the preceding claims, **characterized in that** taurine is used via oral route jointly with said association.

4. The use according to any of the preceding claims, **characterized in that** vitamin D3 is used via oral route jointly with said association.

5. The use according to any of the preceding claims, **characterized in that** the active agents are used in the form of a food supplement.

6. The use according to claim 5 wherein the food supplement also comprises at least one vitamin selected from among vitamin B1, B5, B6, B8, B9, B12, C, D, preferably D3, PP, or vitamin E and derivatives thereof, and preferably comprises vitamin D and/or vitamin E or one of the derivatives thereof, preferably vitamin D3 and/or tocopherol acetate, more preferably vitamin D3 and tocopherol acetate.

7. The use according to any of claims 5 or 6, wherein the food supplement also comprises at least one amino acid selected from among constituent and/or non-constituent amino acids of proteins, in particular arginine, cysteine and/or methionine.

8. The use according to any of claims 5 to 7 wherein the food supplement comprises an association of petroselinic acid, taurine and zinc, and in particular Zn (II) salts, and preferably complexed with one or more polyhydroxy acids such as zinc gluconate.

9. The use according to any of claims 5 to 8 wherein the food supplement comprises petroselinic acid, zinc and in particular a Zn (II) salt, and preferably complexed with one or more polyhydroxy acids, preferably zinc gluconate, taurine, vitamin D3 and tocopherol acetate.

10. Food supplement containing an association of petroselinic acid, zinc in particular a Zn(II) salt, preferably complexed with one or more polyhydroxy acids such as zinc gluconate, and taurine, wherein the petroselinic acid is contained in an amount of between 20 and 70 % by weight relative to the total weight of said association of active agents.

11. The food supplement according to the preceding claim, further comprising vitamin D3 and tocopherol acetate.

12. The food supplement according to claim 10 or 11, comprising:

(i) petroselinic acid in a content of between 1 and 70 weight %, in particular between 10 and 70 weight %, more particularly between 15 and 70 weight % relative to the total weight of the supplement;
(ii) at least one zinc polyhydroxy acid, preferably zinc gluconate in a content of between 0.001 and 30 weight %, in particular between 0.01 and 25 weight %, more particularly between 0.1 and 20 weight % relative to the total weight of the supplement;
(iii) taurine in a content of between 1 and 40 weight %, in particular between 5 and 40 weight %, more particularly between 5 and 30 weight % relative to the total weight of the supplement; and/or
(iv) optionally, vitamin D3 in a content of between 0.0001 and 1 weight %, in particular between 0.0001 and 0.5 weight %, more particularly between 0.0001 and 0.1 weight % relative to the total weight of the supplement; and/or
(v) optionally, tocopherol acetate in a content of between 0.01 and 10 weight %, in particular between 0.1 and 10 weight %, more particularly between 0.2 and 5 weight % relative to the total weight of the supplement.

13. Cosmetic method to prevent and/or combat low-noise microinflammation of hair follicles and/or the scalp, allowing an improvement in hair quality in particular in men over the age of 30 years or in women, comprising the administration to an individual via oral route of the active agents such as defined in any of claims 1 to 9, or of a food supplement such as defined in any of claims 10 to 12.

14. Food supplement comprising one part of the active agents such as defined in claims 1 to 9 in a first composition, and at least the other part of the active agents in a second composition, as a kit or a combination product for simultaneous, separate or time-extended use.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008071897 A

**Littérature non-brevet citée dans la description**

- **MAHÉ et al.** *Skin Pharmacol,* 1996, 366-375
- **MAHÉ et al.** Androgenetic alopecia and micro inflammation. *Int. J. Dermatol.,* 2000, vol. 39, 576-584
- **TRUEB.** *Clinical intervention in aging hair,* 2006, vol. 1 (2), 121-129
- **MAHÉ et al.** *International Journal of Dermatology,* 2000, vol. 39, 576-584
- **AVATO et al.** *Lipids,* 2001, vol. 36, 845